Europäisches Patentamt

European Patent Office

Office européen des brevets

(11) Publication number: **0 412 688 A1**

# EUROPEAN PATENT APPLICATION

(21) Application number: 90308236.0

(22) Date of filing: 27.07.90

(51) Int. Cl.⁵: **C12N 15/52**, C12P 19/40,
C12N 1/21, //(C12N1/21,
C12R1:07),(C12N1/21,
C12R1:125)

The applicant has filed a statement in accordance with Rule 28 (4) EPC (issue of a sample only to an expert). Accession number(s) of the deposit(s): IFO: 14187, 14902, 15040, 14899, 14900, 14901, 14459, FERM: BP-1327, BP-2536,BP-2937, BP-2533, BP-2534, BP-2535, BP-2937, BP-1942.

The applicant has subsequently filed a sequence listing and declared, that it includes no new matter.

(71) Applicant: TAKEDA CHEMICAL INDUSTRIES, LTD.
3-6, Doshomachi 2-chome Chuo-ku
Osaka 541(JP)

(72) Inventor: Miyagawa, Kenichiro
6-11, Higashitokiwadai 6-chome, Toyono-cho
Toyono-gun, Osaka 663-01(JP)
Inventor: Kanzaki, Naoyuki
17-201I, 8 Kuragauchi 2-chome
Ibaraki, Osaka 567(JP)

(74) Representative: Laredo, Jack Joseph et al
Elkington and Fife Beacon House 113
Kingsway
London, WC2B 6PP(GB)

(30) Priority: 04.08.89 JP 204140/89

(43) Date of publication of application:
13.02.91 Bulletin 91/07

(84) Designated Contracting States:
AT BE CH DE DK ES FR GB GR IT LI LU NL SE

(54) **Modified DNA and its use.**

(57) Modified chromosomal DNA containing a DNA sequence responsible for expression of purine operon of a microorganism belonging to the genus Bacillus and DNA of the 3'-adjacent region of the sequence, a part or all of the DNA sequence responsible for expression of purine operon being modified so as to increase the expression is disclosed. A vector integrated with the modified DNA, a novel microorganism belonging to the genus Bacillus being capable of producing inosine and/or guanosine transformed with the DNA or vector as well as an improved process for producing inosine and/or guanosine using the microorganism are also disclosed.

EP 0 412 688 A1

## MODIFIED DNA AND ITS USE

### FIELD OF THE INVENTION

The present invention relates to an improved process for producing inosine and guanosine which are substances of importance as raw materials for the production of 5′-inosinic acid and 5′-guanylic acid, as well as novel microorganisms to be used for the process, and novel DNA to be used for the derivation of the novel microorganisms.

### BACKGROUND OF THE INVENTION

For the production of inosine and guanosine, it is considered that fermentative production using microorganisms is advantageous, and various processes have been proposed and carried out.

As a result of various studies on nucleic acid fermentations to date it has been known that the biosynthesis of a group of substances generally referred to as purine nucleotide related substances such as inosine, guanosine, xanthosine, adenosine as well as their bases (e.g., hypoxanthine) and phosphorylated compounds (e.g., 5′-inosinic acid) undergoes negative feedback regulation by end products such as adenine related substances (e.g., adenosine or its phosphorylated compound such as 5′-adenylic acid) or guanine related substances (e.g., guanosine or its phosphorylated compound such as 5′-guanylic acid).

Therefore, usually, in the fermentative production of a purine nucleotide related substance, the amount of an adenine or its related substances in a culture medium is limited to a low concentration to avoid the regulation of the biosynthetic pathway of the purine nucleotide related substance by the adenine related substance and, further, an adenine-requiring mutant which lacks adenylosuccinate synthetase is used (Y. Nakao, Microbial Technology, Vol. 1, p311-354, edited by H. J. Peppler and D. Perlman, Academic Press, New York, U.S.A.). Furthermore, it has been known to employ processes using bacteria belonging to the genera Bacillus (U.S. Patent No. 3,960,661, Japanese Patent Publication No. 41915/1982, U.S. Patent No. 4,701,413, U.S. Patent No. 3,912,587 and U.S. Patent No. 4,283,346) and Brevibacterium (U.S. Patent No. 3,736,228 and Japanese Patent Publication No. 17592/1983) provided with resistance to various drugs such as the so-called nucleic acid analogues and the like, in addition to an adenine-requiring character to advantageously produce inosine and/or guanosine.

Conventional methods for deriving these mutants are methods wherein mutagenesis treatments such as ultraviolet-light irradiation, treatment with nitrosoguanidine (NTG) and the like are conducted and desired mutants grown on suitable selection media are isolated.

However, since purine nucleotide related substances are the nucleotides responsible for the most important genetic information of organisms, there are sophisticated regulation mechanisms controlling the biosynthesis of the purine nucleotide related substances. As such inosine and/or guanosine are not always readily produced in excess amounts. Furthermore, mutants derived by conventional methods are not always satisfactory because mutation of the desired genes coding for enzymes of purine nucleotide biosynthesis is liable to be accompanied by undesirable mutations in other sites of the chromosomes caused by mutagenesis treatments. Therefore, in view of the industrial importance of purine related substances, it is desired to develop a more improved production method thereof.

### OBJECTS OF THE INVENTION

One object of the present invention is to provide an improved process for producing inosine and/or guanosine.

Another object of the present invention is to provide a novel microorganism belonging to the genus Bacillus to be used for the process of the present invention.

Still another object of the present invention is to provide novel chromosomal DNA which contains the control sequence for expression of a purine operon of a microoganism belonging to the genus Bacillus and the 3′-adjacent region of the sequence, and which is altered to be used for derivation of the novel microorganism of the present invention.

These objects as well as other objects and advantages of the present invention will be apparent to those skilled in the art from the following description with reference to the attached drawings.

## BRIEF EXPLANATION OF THE DRAWINGS

Fig. 1 shows a restriction cleavage map of the recombinant plasmid pPEC32 containing a gene (purE) coding for an enzyme of the purine biosynthetic pathway obtained in Example 1 ii) hereinafter. In Fig. 1, the solid line represents the inserted DNA fragment containing purE. The blank part represents pBR322.

Fig. 2 shows restriction cleavage maps of pPEC32 and pUH32 which contains 5'-upstream region of purE obtained in Example 1 iii) hereinafter. In Fig. 2, the dotted line represents the site cleaved by the same restriction enzyme.

Fig. 3 shows the nucleotide sequence determined in Example 1 iv) hereinafter of Eco RV - Sph I fragment of pUH32 wherein the promoter and the initiation codon of purE are illustrated.

Fig. 4 shows a restriction cleavage map of the plasmid pBX118-9 constructed in Example 1 v) which lacks the control region of purine operon, and a part of the nucleotide sequence thereof is shown in the same figure. In Fig. 4, the solid line represents the DNA fragment containing purE and the blank part represents pUC118. The nucleotide sequence from Kpn I of multi-cloning site to the initiation codon of purE is shown.

Fig. 5 shows the nucleotide sequence of SP promoter prepared in Example 1 vii) hereinafter.

Fig. 6 shows the restriction cleavage map of the recombinant plasmid pBXSC19 constructed in Example 1 viii). The black colored part represents SP promoter, the shaded part represents chloramphenicol-acetyltransferase gene, the solid line part represents the DNA fragment containing purE and the blank part represents pUC19.

Fig. 7 shows the restriction cleavage map of the recombinant plasmid pSB22 constructed in Example 3 hereinafter, and a part of the nucleotide sequence thereof. The blank part represents pUC118.

Fig. 8 shows the restriction cleavage map of the recombinant plasmid pBEI203 constructed in Example 3 hereinafter, and a part of the nucleotide sequence thereof. The blank part represents pUC118.

Fig. 9 shows the restriction cleavage map of the recombinant plasmid pDT32 constructed in Example 3 hereinafter. The blank part represents pBR322 and the shaded part represents chloramphenicol-acetyltransferase gene.

## SUMMARY OF THE INVENTION

The present inventors have intensively studied in order to derive microbial strains wherein repression of a gene for purine nucleotide biosynthesis (purine operon) by adenine related substances and guanine related substances is reduced. As a result, the present inventors have succeeded in the construction of DNA, wherein a DNA region concerned with expression of a purine operon is modified so as to increase expression of the purine operon, and/or the promoter region is replaced with a suitably selected different promoter, by using recombinant DNA techniques, and have also succeeded in the derivation of a novel microorganism, wherein expression of enzymes responsible for the biosynthesis of purine nucleotides is not repressed by adenine related substances and/or guanine related substances. It has further been found that the novel microorganism derived from a recipient, a microorganism belonging to the genus Bacillus , by using this technique can constantly accumulate a large amount of inosine and/or guanosine.

The present invention has been accomplished based on the above findings, and is to provide chromosomal DNA comprising a DNA sequence for expression of a purine operon of a microorganism belonging to the genus Bacillus and DNA of the 3'-adjacent region of the sequence, a part or all of said DNA sequence being modified so as to increase the expression. Further, the present invention also provides a vector containing the modified DNA, a novel microorganism belonging to the genus Bacillus being capable of producing inosine and/or guanosine which is transformed with the DNA or vector of the present invention, and a process for producing inosine and/or guanosine which comprises cultivating the microorganism of the genus Bacillus of the present invention in a culture medium to produce and accumulate inosine and/or guanosine and collecting the resultant.

## DETAILED DESCRIPTION OF THE INVENTION

The term "modified" or "modification" with respect to increase in purine operon expression used herein includes a deletion of a part of a DNA sequence responsible for expression of a purine operon, insertion of different DNA into the sequence and/or replacement of a part or all of the sequence with different DNA.

DNA containing the control region for expression of purine operon used in the present invention does not necessarily contain the whole region of purine operon, but it contains a promoter sequence, a DNA sequence concerned with expression of purine operon regulated by one or more adenine related substances such as adenine, adenosine, adenosine monophosphate and adenosine diphosphate, adenosine triphosphate and that concerned with expression regulated by one or more guanine related substances such as guanine, guanosine, guanosine monophosphate, guanosine diphosphate and guanosine triphosphate, which are located in the neighbourhood of the promoter sequence, as well as the $3'$-adjacent region downstream therefrom. More preferably, the DNA further contains some $5'$-adjacent region. Such a DNA is that which contains at least 0.1 kilo base pair (kbp) of the $3'$- downstream region and about 0.4 kbp of the $5'$-upstream region from the N-terminal end of the first open reading frame in a purine operon. Hereinafter, this part is referred to as a region relating to regulation of purine operon expression.

Such a DNA is conveniently isolated from chromosomal DNA of a microorganism by using recombinant DNA techniques. As a host-vector system for this purpose, EK system which is commonly employed can be suitably used. That is, as a host, there can be suitably used strains derived from Escherichia coli K-12 strain, for example, Escherichia coli C600 (T. Maniatis et al., Molecular Cloning, A Laboratory Manual, Cold Spring Harbor Laboratory, published by Cold Spring Harbor Laboratory Press, 1982, page 504), Escherichia coli JA221 (IFO 14359, ATCC 33875), Escherichia coli MM294 (ATCC 33625) [Proc. Natl. Acad. Sci. USA, 73, 4174 (1976)] or derivatives thereof. As a vector, there can be suitably used pBR322, pACYC184, pACYC177, pUC18, pUC19, pHSG396, pHSG298 and the like.

Further, other host-vector systems such as combinations of phage vectors, for example, Charon 4A (Molecular Cloning, page 31), EMBL 3 and EMBL 4 (J. Mol. Biol., 170, 827) and the like, to Escherichia coli DP50 supF (Molecular Cloning, page 504), Escherichia coli NM 538 and NM 539 (J. Mol. Biol., 170, 827), Escherichia coli LE 392 (J. Biol. Chem., 218, 97) and the like can be used. Of course, a host-vector system wherein a microbe of the genus Bacillus, for example, Bacillus subtilis MI 114 (Gene, 24, 255) or its mutant is used as the host and a plasmid having the capability of autonomous replication in a microorganism of the genus Bacillus such as pUB110, pC194, pE194, pS194, pSA2100, pHY300PLK or the like is used as the vector can also be employed.

In principle, the origin of a donor of DNA containing the region relating to regulation of purine operon expression is not specifically limited in so far as the nucleotide sequence of the DNA of the donor has high homology to that of the region on the chromosome of a microorganism of the genus Bacillus to be used as a recipient as described hereinafter, and any microorganism can be used as the donor. Among them, when a microorganism of the genus Bacillus is used, so-called self cloning is realized and it is preferred because of easy handling. Further, the resulting transformant is expected to be stable. Furthermore, the donor is not necessarily capable of producing inosine, guanosine, xanthosine or purine bases thereof.

Examples of such a DNA donor include Bacillus subtilis, Bacillus pumilus or Bacillus licheniformis. For example, the following microbial strains can be used: Bacillus subtilis No. 168 (BGSC 1A1), Bacillus subtilis No. 115 (IFO 14187, FERM BP-1327), Bacillus subtilis MI 114 (Gene, 24, 255), Bacillus pumilus ATCC 7061 and Bacillus licheniformis ATCC 14580 (wherein BGSC is The Bacillus Genetic Stock Center, IFO is Institute of Fermentation, Osaka and ATCC is The American Type Culture Collection).

The chromosomal DNA can be isolated from these donors according to the conventional methods, for example, extraction of chromosomal DNA with phenol (H. Sato and K. Miura, Biochim. Biophy. Acta., 72, 619). The chromosomal DNA thus obtained is cleaved with a suitably selected restriction enzyme and ligated to a vector with a DNA ligase. The ligation mixture is used for transformation of a host having a deficiency in an enzyme of purine nucleotide biosynthesis which has been coded on purine operon, to select a transformant wherein the deficiency has been complemented. The transformation of the host can be carried out according to the conventional methods. For example, in the case with Escherichia coli, the transformation can be carried out according to the method described by S.N. Cohen et al. [Proc. Natl. Acad. Sci. USA, 69, 2110). In the case that the host is a strain of Bacillus subtilis, the transformation can be carried out according to the method described by S. Chang and S.N. Cohen (Molec. Gen. Genet., 168, 115).

Whether the deficiency in an enzyme of purine nucleotide biosynthesis of the transformant thus obtained is complemented or not can be readily determined by its growth on a culture medium with or without purine nucleotide related substance and, if necessary, containing required nutrients other than purine nucleotide related substances.

Thus, whether DNA containing the region relating to regulation of purine operon expression is present in a cloned DNA fragment or not can be determined by comparing the nucleotide sequence, restriction cleavage sites and the like with those of purine operon of a known Bacillus subtilis strain [J. Biol. Chem., Vol. 262, pp 8274-8287 (1987)], or can be determined by using a promoter probe vector (J. Bacteriol., Vol. 146, pp 1162-1165). Further, DNA containing the region relating to regulation of purine operon expression can also be cloned by using colony hybridization or plaque hybridization (Molecular Cloning, pages 312 to 328) based on the nucleotide sequence data of purine operon of a known Bacillus subtilis strain. A large amount of DNA containing the gene from the transformant can be prepared according to the method described in the above "Molecular Cloning", pages 86 to 93.

For the construction and isolation of the novel DNA of the present invention from the DNA containing the region relating to regulation of purine operon expression thus obtained, recombinant DNA techniques are conveniently employed and, for example, a host-vector system of Escherichia coli can be suitably used.

In the following construction steps of the DNA of the present invention in Escherichia coli , in many cases, it is convenient to subclone a DNA fragment in a new vector or to ligate it by using a polylinker. For this purpose, it is preferable to carry out subcloning by utilizing, for example, a polylinker portion of pUC118 and pUC119 which are vectors of Escherichia coli because both the construction of the novel DNA and the determination of nucleotide sequence can be conveniently carried out.

The novel DNA of the present invention can be constructed and isolated from a plasmid containing the region relating to regulation of purine operon expression by using a suitable restriction enzyme and, if necessary, an enzyme having exonuclease activity which digests a double stranded DNA from its end in order, for example, BAL31 to obtain a shortened DNA fragment wherein the region relating to regulation of purine operon expression is deleted. In this case, for obtaining DNA fragments wherein each of the nucleotide sequence responsible for repression of a purine operon by adenine related substances, purine operon promoter and the nucleotide sequence responsible for repression of purine operon by guanine related substances is deleted, the desired DNA fragment can be selected by varying an amount of the enzyme used and a reaction time to obtain various DNA fragments having different lengths and different kinds of deletion, and selecting the desired one by suitable means as described hereinafter, for example, according to the results of nucleotide sequence determination of the fragments. The determination of nucleotide sequence can be carried out by subcloning the DNA fragment in a vector for the determination of nucleotide sequence, for example, M13, pUC118 or pUC119, and then using a known method, for example, the method described by F. Sanger et al. (Proc. Natl. Acad. Sci. USA, 74 , 5463).

According to this method, it is possible not only to remove the region responsible for repression of a purine operon by adenine related substances and/or that region responsible for repression of a purine operon by guanine related substances, but also to delete the promoter region to replace it with a suitable selected different promoter. For this purpose, a DNA fragment wherein the promoter region of purine operon has been deleted may be ligated downstream from a promoter sequence expressible in a microorganism belonging to the genus Bacillus with, for example, T4 DNA ligase. In this case, if a marker gene which can be expressed in a microorganism belonging to the genus Bacillus , for example, a drug resistant gene, is ligated thereto, the selection of a transformant belonging to the genus Bacillus as described hereinafter may be conveniently carried out.

Further, in this case, a part of the 5′-adjacent region of the promoter of a purine operon is ligated to the 5′-upstream region from the ligated promoter. This is convenient because double crossing-over type recombination is carried out on chromosomes of a recipient as described hereinafter.

The promoter used in the present invention is not limited to a specific one in so far as it has a DNA sequence which can be expressed in a recipient to be used in the later step, regardless of its origin, i.e., prokaryote, eukaryote or synthetic DNA. Examples thereof include promoters derived from chromosomes of microorganisms belonging to the genus Bacillus , phage of microorganisms belonging to the genus Bacillus , or plasmids having the capability of autonomous growth in cells of microorganisms belonging to the genus Bacillus . Of course, the promoter may be that of a purine operon of a microorganism belonging to the genus Bacillus . In the latter case, the modification is limited to the control region regulated by adenine related substances and/or the control region regulated by guanine related substances. Specific examples of such promoters are as follows:

AAAAGGTA TTGACT TTCCCTACAGGGTGTGT AATAAT TTATATACA

SPO1-15 [Lee, G and Pero, J, J. Mol Biol., 152 247 (1981)]

AAAAGTTG TTGACT TTATCTACAAGGTGTGG CATAAT AATCTTAAC

SPO1-26 [Lee et al., Mol. Gen. Genet., 180 , 57 (1980)]

GAAAAGTG TTGAAA ATTGTCGAACAGGGTGA TATAAT AAAAGAGTA

EP 0 412 688 A1

φ29G3b [Marray, C.L. and Robinowtiz, J.C., J. Biol. Chem., 257 , 1053 (1982)]
GAAAAGGG TAGACA AACTATCGTTTAACATGT TATACT ATAATAGAA
φ29G2 [Yoshikawa, H. et al., Proc. Natl. Acad. Sci. USA, 78 , 1336 (1981)]
ATTAATGT TTGACA ACTATTACAGAGTATGC TATAAT GGTAGTATC
φ29A1 [Yoshikawa, H. et al., Proc. Natl. Acad. Sci. USA, 78 , 1336 (1981)]

Such a promoter can be cut out from DNA (plasmid) containing the appropriate promoter with a suitable restriction enzyme, fractionated by, for example, agarose gel electrophoresis or polyacrylamide gel electrophoresis, and then recovered. Alternatively, DNA having the nucleotide sequence of the above promoter can be synthesized according to the phosphoamidate method by using a commercially available DNA synthesizer (e.g., the apparatus manufactured by Allied Biosystems Inc., U.S.A.) and adhering to its protocol.

In order to obtain a large amount of the novel DNA of the present invention, a host microorganism is transformed with the above ligation mixture, followed by collecting a transformant grown on a selective medium, for example, in the case of utilizing drug resistance as a selection marker, a medium containing the corresponding drug. A large amount of the DNA can be prepared from the transformant according to a method which itself has been known such as the method described in pages 86 to 93 of the above "Molecular Cloning".

In order to isolate a DNA fragment containing the modified region relating to regulation of purine operon expression from the plasmid thus obtained, the plasmid is cleaved with a restriction enzyme according to the conventional method, followed by separating with agarose gel electrophoresis and then extracting and isolating with, for example, an electro-eluter.

Hereinafter, a method for the derivation of the novel microorganism by transformation of a recipient with the novel DNA thus obtained is illustrated.

As the recipient for this transformation, any microorganism having capability of transformation, i.e., a property such as DNA provided outside of the cells can be incorporated therein and recombination is caused at a part on its chromosome having high homology to the nucleotide sequence of the DNA to change its inherent character, can be used. The microorganism is not necessarily identical with the donor of the region relating to regulation of purine operon expression in so far as the nucleotide sequence of 3'-downstream region of the region relating to regulation of purine operon expression in the chromosomal DNA has high homology to that of 3'-downstream region of the region relating to regulation of purine operon expression contained in the outside DNA. For the derivation of a transformant having high capability of producing inosine and/or guanosine, microorganisms belonging to the genus Bacillus such as Bacillus subtilis , Bacillus pumilus , Bacillus licheniformis and the like are suitable from the viewpoints that they are known to accumulate two or more purine nucleotide related substances such as inosine, guanosine and xanthosine, they are capable of transformation, they have no pathogenicity, and they have a history that they have been used safely in fermentation industries.

Further, in many cases, a much improved result in the production of inosine and/or guanosine can be obtained, when a microorganism wherein one or more known characteristics advantageous for accumulation of purine nucleotide relating substances, for example, purine analogue resistance such as 8-azaguanine resistance, nucleoside phosphorylase deficiency, 5'-guanylic acid reductase deficiency, folic acid antagonist resistance are provided by the above microorganism is used as the recipient.

Examples of the recipient of the genus Bacillus include:
Bacillus subtilis BM 1032 (IFO 14559, FERM BP-1242)
Bacillus subtilis 1A3 (BGSC No. 1A3)
Bacillus subtilis NA-6011 (IFO 14189, FERM BP-291)
Bacillus subtilis NA-6012 (IFO 14190, FERM BP-292)
Bacillus subtilis NA-6128 (IFO 14373, FERM BP-617)
Bacillus subtilis NA-7821 (IFO 14368, FERM BP-618)
Bacillus subtilis ATCC 19221
Bacillus subtilis ATCC 14662
Bacillus pumilus (FERM P-2116)
Bacillus pumilus (FERM P-4832)
Bacillus pumilus (FERM P-4829)
Bacillus licheniformis IFO 12485

The novel DNA of the present invention is used for transformation of a microorganism belonging to the genus Bacillus and, in this case, it is possible to use it in an integrated form in a plasmid, or in the form of a linear DNA obtained by cleavage of a plasmid or cutting out of the DNA of the present invention. The transformation of a microorganism of the genus Bacillus with the DNA fragment can be carried out

6

according to a known method [C. Anagnostopoulos and J. Spizizen, J. Bacteriol., 81 , 741 (1961); etc.].

The selection of the transformant can be readily carried out by, for example, in the case of using a drug resistant gene as a selection marker, cultivation on an agar plate containing the corresponding drug. Whether the transformant thus obtained is the novel microorganism having the desired characteristics or not can be readily determined by measuring the activity of an enzyme of purine nucleotide biosynthesis system coded by the purine operon. For example, in the transformant of the genus Bacillus which has been transformed by DNA of the present invention wherein the region concerned with repression by adenine related substances has been modified, high enzymatic activity is maintained because an enzyme of purine nucleotide biosynthesis (e.g., PRPP amidotransferase which is the first enzyme of the biosynthesis) is not repressed even if an excess amount of adenine is added to a culture broth and there is a great difference in the activity between the transformant and the recipient. Thereby, the transformant is readily determined to be the novel microorganism.

Typical examples of the transformant of the present invention include Bacillus subtilis BM2032 (IFO 14902, FERM BP-2536 or Bacillus subtilis BM2232 (IFO 15040, FERM BP-2937)) obtained in the Examples hereinafter.

Of course, by selecting the promoter and the recipient, various transformants can be readily derived according to the method as described herein.

In order to produce inosine and/or guanosine by using the transformant obtained by the present invention, a conventional fermentation process for producing inosine and/or guanosine can be employed.

That is, culture media containing carbon sources, nitrogen sources, metal ions and, if necessary, other nutrients such as amino acids, nucleic acids, vitamins and the like can be used. As the carbon sources, glucose, sucrose, maltose, starch, saccharified starch, molasses and the like can be used. They can be used alone or in combination thereof. As the nitrogen sources, in addition to organic nitrogen sources such as peptone, soybean meal, dried yeast, urea and the like, inorganic nitrogen sources such as ammonium salts of sulfuric acid, nitric acid, hydrochloric acid, carbonic acid and the like, gaseous ammonia, aqueous ammonia and the like can also be used alone or in combination thereof. As other nutrients, various inorganic salts, amino acids, vitamins and the like which are necessary for the growth of the microorganism are appropriately selected and used alone or in combination thereof. As adenine sources, adenine, adenosine and adenylic acid as well as microbial cells containing them and their extract and the like can be used.

Further, if necessary, an antifoaming agent such as silicone oil, polyalkylene glycol ether or the like, a surfactant and the like can be added to the culture medium.

Usually, the cultivation is carried out under aerobic conditions such as shaking, aeration submerged culture and the like. Preferably, pH of the culture medium is within the range of 4 to 9. When pH is varied during cultivation, sulfuric acid, calcium carbonate, sodium hydroxide, gaseous ammonia, aqueous ammonia and the like can be added to adjust pH to the above preferred range. Usually, the cultivation temperature is appropriately selected from the range of 20 to 45°C so that the temperature is suitable for the growth of the microorganism to be used and for the accumulation of inosine and/or guanosine. The cultivation is continued until the accumulation of inosine and/or guanosine substantially becomes maximum. Usually, the desired result can be obtained by cultivation for 24 to 144 hours.

Known purification means, for example, precipitation, and isolation and purification methods such as chromatography using an ion exchange resin, activated charcoal and the like can be employed to isolate and obtain inosine and/or guanosine.

According to the present invention, the industrially advantageous production of inosine and/or guanosine which are the starting materials for the production of important flavor substances, 5′-inosinic acid and 5′-guanylic acid can be realized with high yield.

That is, a novel microorganism having an improved enzymatic activity of purine nucleotide biosynthesis system can be obtained by using a microorganism of the genus Bacillus being capable of producing inosine and/or guanosine as a recipient and transforming it with the DNA of the present invention to release repression by adenine related substances and guanine related substances, and/or to replace a promoter with a different promoter. The novel microorganism can very stably produce a large amount of inosine and/or guanosine.

The following Examples further illustrate the present invention in detail but are not to be construed to limit the scope thereof.

The operations in the Examples were carried out according to the following methods (1) to (9) unless otherwise stated.

(1) Digestion of DNA with restriction enzyme

7

Buffer solutions for restriction enzymes (manufactured by Takara Shuzo Co., Ltd., Japan) used were those specified by the manufacturer. The digestion was carried out by using the enzyme at a concentration of 10 units/ug DNA at 37°C for 60 minutes. Then, the reaction mixture was extracted with phenol saturated with TE buffer [10 mM Tris-HCl (pH 7.5) and 1 mM EDTA]. To the extract were added 1/10 volume of 3 M sodium acetate (pH 6) and then 2.5 volumes of ethanol and the mixture was centrifuged to recover DNA.

(2) Extraction of a large amount of plasmid

The extraction was carried out according to the method described at pages 86 to 93 of "Molecular Cloning". Namely, Escherichia coli harbouring plasmid DNA was inoculated to LB medium (250 ml; Bacto tryptone 10 g/liter, yeast extract 5 g/liter and sodium chloride 5 g/liter). After overnight cultivation, the cells were collected and washed. Then, lysozyme was added to the washed cells and further 0.2 N sodium hydroxide solution containing 1% sodium lauryl sulfate was added to cause lysis of the cells. After addition of 5 M potassium acetate, a supernatant containing the plasmid was obtained by centrifugation. To the supernatant was added 0.6 volume of isopropanol to precipitate the plasmid DNA and, after washing with ethanol, it was dissolved in TE buffer. To the solution was added cesium chloride so that the specific gravity became 1.60 and further added ethidium bromide so that the final concentration became 600 $\mu$g/ml. The mixture was centrifuged at 50,000 rpm at 20°C for 12 hours by an ultracentrifuge (rotor: $V_{65}Ti$) and a band of the plasmid detected by ultraviolet rays was taken out. After removing ethidium bromide with n-butanol, ethanol precipitation was conducted.

(3) Transformation of Escherichia coli

The transformation was carried out according to the method described at page 250 of "Molecular Cloning".

Namely, Escherichia coli was inoculated to LB medium (3 ml). After overnight cultivation, a 1 ml portion thereof was inoculated to LB medium (100 ml) and incubated with shaking at 37°C until the cell concentration became about 5 x $10^7$ cells/ml. Then, the cells were collected, and they were suspended and ice-cooled for 15 minutes by addition of ice-cooled sterilized water (50 ml) containing 50 mM calcium chloride and 10 mM Tris-HCl buffer (pH 8). The cells were separated by centrifugation and they were again suspended in sterilized water (6.7 ml) containing 50 mM calcium chloride and 10 mM Tris-HCl buffer. To a 2 ml portion thereof was added DNA and the cell suspension was ice-cooled for 30 minutes. LB medium (0.8 ml) was added thereto. The cell suspension was incubated at 37°C for 1 hour, spread on a LB agar plate containing a drug and further incubated at 37°C overnight.

(4) BAL31 digestion treatment

BAL31 nuclease digestion treatment was carried out to digest both double strands of DNA from both terminal ends. Namely, DNA (10 $\mu$g) treated with a restriction enzyme was dissolved in BAL3I buffer solution [100 $\mu$l; 12 mM calcium chloride, 12 mM magnesium chloride, 200 mM sodium chloride and 20 mM Tris-HCl (pH 8), 2 mM EDTA] and BAL31 nuclease (1 unite, manufactured by Takara Shuzo Co., Ltd., Japan) was added thereto. The reaction mixture was incubated at 30°C. After digestion, phenol extraction and ethanol precipitation were conducted. Then, in order to convert the digested terminal ends of DNA into blunt ends, the above DNA was suspended in a buffer solution for T4 DNA polymerase [20 $\mu$l; containing 33 mM Tris-acetic acid (pH 7.9), 10 mM magnesium acetate, 0.5 mM dithiothreitol, 66 mM potassium acetate, 0.01% BAS, 0.1 mM dATP, 0.1 mM dGTP, 0.1 mM dCTP and 0.1 mM d TTP] and T4 DNA polymerase (5 units, manufactured Takara Shuzo Co., Ltd., Japan) was added thereto. The reaction mixture was incubated at 37°C for 30 minutes. After incubation, phenol extraction and ethanol precipitation were conducted.

(5) Ligation of DNA

A DNA ligation kit (manufactured by Takara Shuzo Co., Ltd., Japan) was used for ligation of 2 to 3 DNA fragments. The ligation conditions were followed to those specified by the manufacturer.

(6) Transformation of Bacillus subtilis

The transformation was carried out according to the method described by Dubnou et al. Namely, a microorganism was inoculated to LB medium (5 ml) and incubated at 37°C overnight. A 0.5 ml portion thereof was transferred to SPI medium (20 ml; containing 1.4% dipotassium phosphate, 0.6% monopotassium phosphate, 0.2% ammonium sulfate, 0.1% sodium citrate, 0.02% magnesium sulfate, 0.5% glucose, 0.02% Casamino acid, 0.1% yeast extract, tryptophane 50 μg/ml and leucine 50 μg/ml) and incubated at 37°C for 4 hours. Then, a 10 ml portion of the latter culture was transferred to SPII medium (100 ml; containing 1.4% dipotassium phosphate, 0.6% monopotassium phosphate, 0.2% ammonium sulfate, 0.1% sodium citrate, 0.02% magnesium sulfate, 0.5% glucose, calcium chloride 75 μg/ml and magnesium chloride 508 μg/ml) and incubated at 37°C for 90 minutes. To a 1 ml portion thereof was added DNA and cell suspension was incubated at 37°C for 30 minutes. Then, the cell suspension was spread on LB agar plate containing a drug and incubated at 37°C overnight.

(7) Determination of PRPP amidotransferase activity

The transformant was inoculated to LB medium (5 ml) and incubated at 37°C overnight. A 0.2 ml portion thereof was inoculated to SPI medium (20 ml) containing adenine or guanine (300 μg/ml) and incubated at 37°C for 3 hours. The cells were collected and washed with 0.85% aqueous sodium chloride solution. Again, the cells were washed with acetone and dried. Then, the cells were suspended in a reaction solution I [1 ml; 100 mM Tris-HCl (pH 7.5), 3.2 mM magnesium chloride, 2.5 mM PRPP and 12 mM L-glutamine] and incubated at 37°C for 15 minutes. The reaction mixture was held in boiling water for 3 minutes to terminate the reaction. The reaction mixture was centrifuged and the concentration of glutamic acid in the supernatant was measured by using NAD and glutamate dehydrogenase (manufacture by Boehringer Mannheim Yamanouchi Co., Ltd., Japan). The activity was represented by nmol glutamic acid produced/min./mg dried cells.

(8) Colony hybridization

The transformed colonies were inoculated on a nylon filter (colony/plaque screen NEF-978X manufactured by E.I. du Pont de Nemours & Co., Inc., U.S.A.) placed on LB agar plate containing ampicillin (50 μg/ml) and on a master plate of LB agar plate containing ampicillin, respectively, and they were incubated at 37°C overnight.

The nylon filter was removed from the plate and dipped in 0.5 N aqueous sodium hydroxide solution for 10 minutes and further in 1 M Tris-HCl (pH 7.5) for 10 minutes. Then, the filter was dried at room temperature.

On the other hand, a probe was prepared. The preparation was carried out by using a multiprime DNA label system (manufactured by Amasham Japan Co., Ltd., Japan) according to the protocol specified by the manufacturer.

The method for hybridization was followed to the protocol for the nylon filter.

(9) Determination of nucleotide sequence

The determination of the nucleotide sequence was carried out by using SEQUENASE (manufactured by Toyo Boseki Kabushiki Kaisha, Japan) according to the method specified by the manufacturer.

The IFO number used herein is the accession number in Institute for Fermentation, Osaka and FERM BP-number is the accession number in Fermentation Research Institute, Agency of Industrial Science and Technology (FRI) under the Budapest treaty.

Example 1

i) Preparation of chromosomal DNA

Bacillus subtilis No. 115 [IFO 14187, FERM BP-1327 (deposited March 28, 1987)] was inoculated to LB medium and incubated at 37° C overnight. Chromosomal DNA (5 mg) was obtained by chromosomal DNA extraction with phenol.

ii) Cloning of purE

The chromosomal DNA (10 μg) obtained in the above i) was digested with Cla I and fractionated by agarose gel electrophoresis and DNA corresponding to about 3.6 to 4.5 kbp was recovered with a unidirectional electro-eluter (manufactured by International Biotechnologies, Inc., U.S.A.).

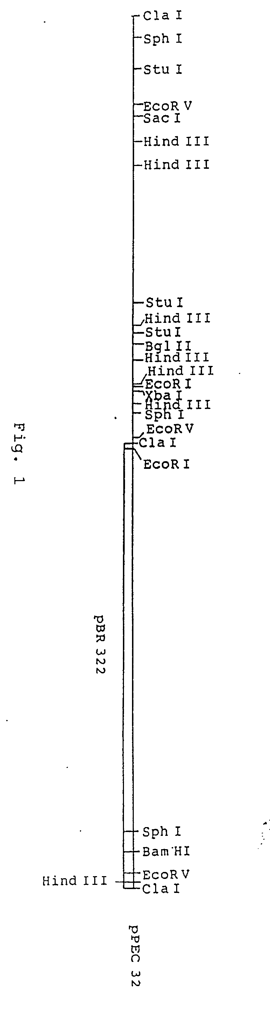

On the other hand, pBR322 (0.5 μg) was digested with Cla I and mixed with the above DNA fraction to begin ligation reaction. The purE deficient strain Escherichia coli PC 0135 (CGSC5403) was transformed with this ligation mixture, spread on a M9CA agar plate (containing disodium phosphate 6 g/liter, monopotassium phosphate 3 g/liter, sodium chloride 0.5 g/liter, ammonium chloride 1 g/liter, glucose 2 g/liter, 2 mM magnesium sulfate, 0.1 mM calcium chloride and Casamino acid 2 g/liter) containing ampicillin, and incubated at 37° C for one day to obtain a transformant, Escherichia coli PC 0135 (pPEC32) [IFO 14899, FERM BP-2533 (deposited July 26, 1989)]. The plasmid pPEC32 was obtained from the transformant. Then, a large amount of pPEC32 was extracted and cleaved with various restriction enzymes. According to its electrophoresis pattern, the restriction cleavage map as shown in Fig. 1 was obtained using Hin dIII digested lambda DNA as a molecular standard.

iii) Preparation of upstream part DNA of purE gene

The chromosomal DNA (10 μg) obtained in the above i) was digested with Hin dIII and fractionated by agarose gel electrophoresis to recover DNA corresponding to about 2.0 to 2.6 kbp.

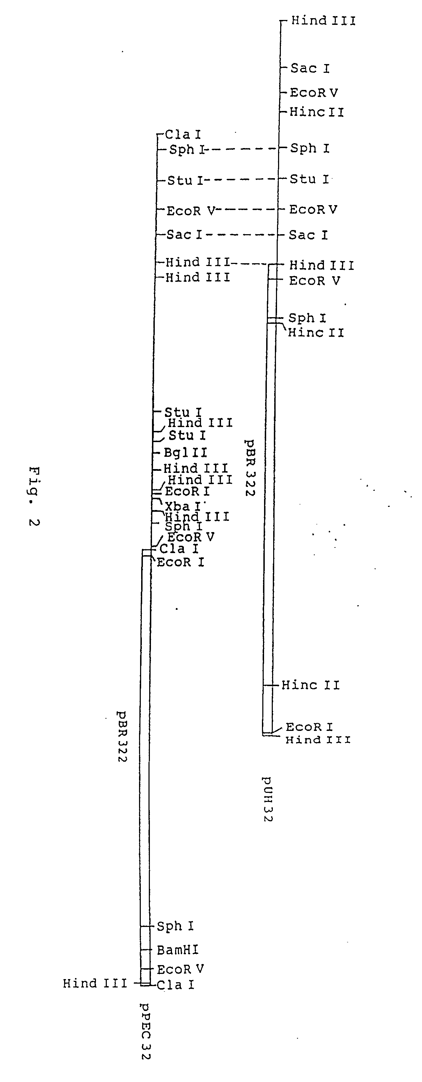

On the other hand, pBR322 (0.5 μg) was digested with Hin dIII, dephosphorylated with alkaline phosphatase and subjected to phenol extraction and ethanol precipitation. This was mixed and ligated with the above DNA fragment. Escherichia coli DH1 (ATCC 33849) was transformed with this ligation mixture and ampicillin resistant strains were selected. About 500 colonies were selected and subjected to colony hybridization by using Cla I-Stu I fraction (620 bp) of pPEC32 as the probe. Among colonies hybridized with the probe, one strain, Escherichia coli DHI (pUH32) [IFO 14900, FERM BP-2534 (deposited July 26, 1989)] was selected to obtain the plasmid pUH32. A large amount of pUH32 was extracted and digested with various restriction enzymes. According to its electrophoresis pattern, the restriction cleavage map as shown in Fig. 2 was obtained.

iv) Determination of nucleotide sequence upstream from purine operon

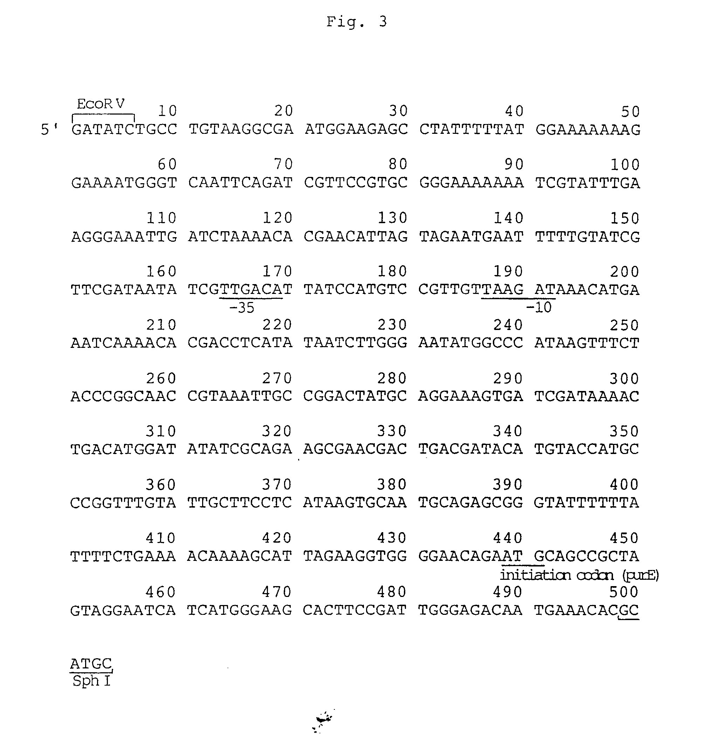

In order to determine the nucleotide sequence of Eco RV-Sph I fragment (about 500 bp) of pUH32 obtained in the above iii), pUH32 (1 μg) was double-digested with Eco RV and Sph I and fractionated by agarose gel electrophoresis to recover a fragment (about 500 bp). Then, this was ligated with pUC119 (0.5 μg) double-digested with Sma I and Sph I, and the ligation mixture was used to transform Escherichia coli JM109 (supplied by Takara Shuzo Co., Ltd., Japan) to obtain ampicillin resistant strains. A plasmid was extracted from one of these strains and the nucleotide sequence thereof was determined. The nucleotide sequence was as shown in Fig. 3. This nucleotide sequence was identical with that reported in Ebbole, D.J. et al., J. Biol. Chem., 262 , 8274-8287.

v) Construction of deficient plasmid pBX118-9

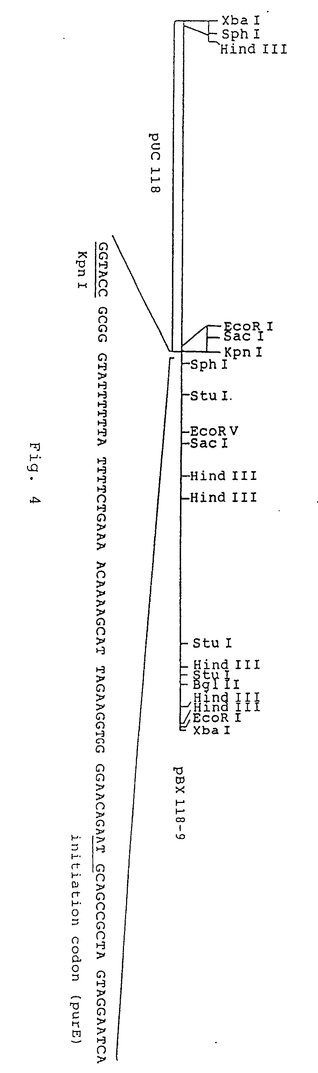

pPEC32 (10 μg) obtained in the above ii) was digested with Cla I and subjected to BAL31 digestion treatment for 30 seconds. Then, it was completely digested with Xba I and fractionated by agarose gel electrophoresis to recover a DNA fragment corresponding to about 3.6 kbp. This DNA was ligated to pUC 118 (0.5 μg) double-digested with Sma I and Xba I and the ligation mixture was used for transformation of Escherichia coli JM109 to select an ampicillin resistant strain. As a result, the plasmid pBX118-9 as shown in Fig. 4 was obtained.

### vi) Construction of pSKC

pCAT15 (0.5 μg, Japanese Patent Laid Open Publication No. 62-82096) was double-digested with Bam HI and Pst I and the digested ends were converted into blunt ends with T4 DNA polymerase. This DNA was ligated to BLUESCRIPT SK (0.5 μg, manufactured by Toyo Boseki Kabushiki Kaisha, Japan) cleaved with Sma I and this was used for transformation of Escherichia coli JM109 to select an ampicillin resistant and chloramphenicol resistant strain. The plasmid pSKC was obtained from this resistant strain.

### vii) Preparation of SP promoter

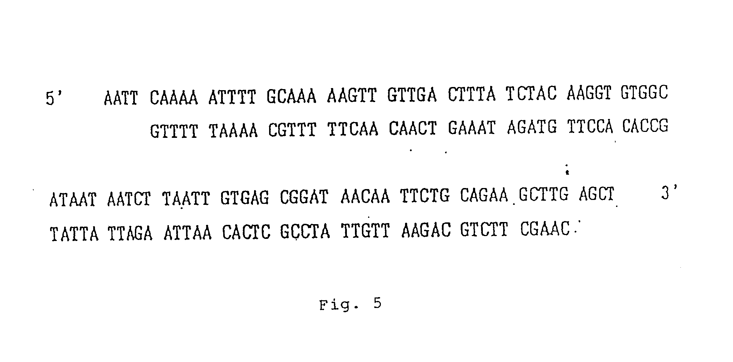

A promoter expressible in Bacillus subtilis (Fig. 5) was prepared by using a DNA synthesizer (DNA synthesizer 381A manufactured by Applied Biosystems Japan Co., Ltd., Japan) according to the protocol specified by the manufacturer. Then, this was ligated to pUC19 (0.5 μg) cleaved with Eco RI and Sac I and used for transformation of Escherichia coli JM109 to obtain the plasmid pSP19 integrated with SP promoter.

### viii) Construction of pBXSC19

pSP19 obtained in the above vii) (0.5 μg) was double-digested with Kpn I and Xba I. On the other hand, pSKC obtained in the above vi) was digested with Xba I and fractionated by agarose gel electrophoresis to obtain cat gene (about 1.0 kbp). Further, pBX118-9 obtained in the above v) was double-digested with Kpn I and Xba I and fractionated by agarose gel electrophoresis to obtain a DNA fragment (about 3.6 kbp).

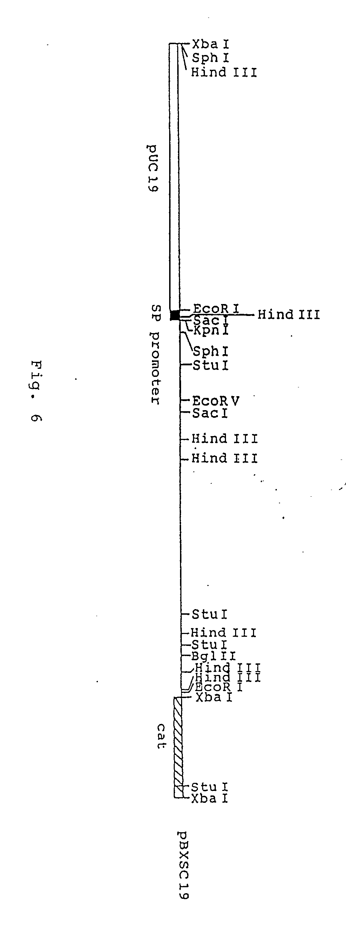

Then, these three fragments were mixed, ligated and used for transformation of Escherichia coli JM109 to select an ampicillin resistant strain, Escherichia coli JM109 (pBXSC19) [IFO 14901, FERM BP-2535 (deposited July 26, 1989)]. As a result, the plasmid pBXSC19 as shown in Fig. 6 was obtained.

### Example 2

#### i) PRPP amidotransferase activity of Bacillus subtilis 168 transformed with pBXSC19

Bacillus subtilis 168 was transformed with pBXSC19 (1 μg) obtained in Example 1 viii) to obtain chloramphenicol resistant strains. One of these strains was cultivated in SPI medium, SPI medium containing adenine and SPI medium containing guanine, and PRPP amidotransferase activities were measured. As control experiments, the activities of Bacillus subtilis 168 were also measured. The results are shown in Table 1.

Table 1

| Strains | SPI | SPI + adenine | SPI + guanine |
|---|---|---|---|
| B . subtilis 168 | 5.4 | 1.4 | 1.0 |
| B . subtilis 168 (pBXSC19) | 18 | 18 | 11 |
| (Units) | | | |

#### ii) Transformation of inosine-guanosine producer Bacillus subtilis BM1032

Bacillus subtilis BM1032 (IFO 14459, FERM BP-1242) was transformed with pBXSC19 (1 μg) obtained in Example 1 viii) to obtain chloramphenicol (10 μg/ml) resistant strains. One of the transformants was named as Bacillus subtilis BM2032 [IFO 14902, FERM BP-2536 (deposited July 26, 1989)].

EP 0 412 688 A1

iii) Inosine and guanosine productivity by Bacillus subtilis BM2032

One loopful of Bacillus subtilis BM2032 was inoculated in LB medium (5 ml) and incubated with shaking at 37°C for 12 hours. A 1 ml portion thereof was transferred to the medium (20 ml) as shown in Table 2 and cultivated at 37°C for 48 hours. After completion of the cultivation, the amount of inosine and guanosine accumulated in the culture broth was determined by high performance liquid chromatography. As a result, 14 mg/ml of inosine and 2 mg/ml of guanosine were accumulated.

On the other hand, Bacillus subtilis BM1032 strain was cultivated under the same conditions as those described above. As a result, only 8.0 mg/ml of inosine and 1.1 mg/ml of guanosine were accumulated.

Table 2

| Components of medium | Concentration (g/liter) |
|---|---|
| Glucose | 120 |
| Sodium glutamate | 12 |
| Ammonium nitrate | 20 |
| DL-$\alpha$-alanine | 0.3 |
| Casamino acid | 10 |
| Tryptophane | 0.2 |
| Leucine | 0.2 |
| RNA | 2 |
| Biotin | 0.0002 |
| Monopotassium phosphate | 2 |
| Potassium chloride | 1 |
| Magnesium sulfate | 1 |
| Ferrous sulfate | 0.005 |
| Manganese sulfate | 0.01 |
| Calcium carbonate | 15 |
| pH | 7.5 |

Example 3

i) Construction of deficient plasmid pSB22

pUH32 (0.5 $\mu$g) obtained in Example 1 iii) was triple-digested with Sac I, Sph I and Stu I and fractionated by agarose gel electrophoresis to recover a fragment containing the promoter (about 800 bp). Then, this DNA was ligated to pUC118 (0.5 $\mu$g) double-digested with Sac I and Sph I and used for transformation of Escherichia coli JM109. An ampicillin resistant strain was selected to obtain the plasmid pUSS118.

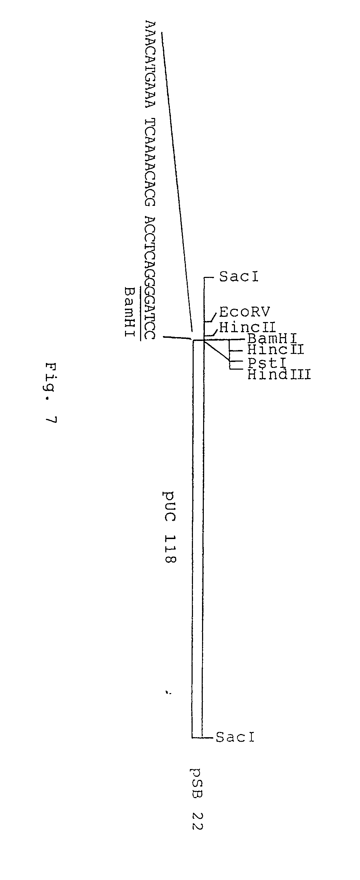

Then, pUSS118 (10 $\mu$g) was digested with Sph I, subjected to BAL31 digestion treatment for 30 seconds, digested with Sac I and fractionated by agarose gel electrophoresis to recover a DNA fragment corresponding to about 500 bp. This DNA was ligated to pUC118 (0.5 $\mu$g) double-digested with Sac I and Sma I and used for transformation of Escherichia coli JM109 to select an ampicillin resistant strain. As a result, the plasmid pSB22 as shown in Fig. 7 was obtained.

ii) Construction of pBEI203

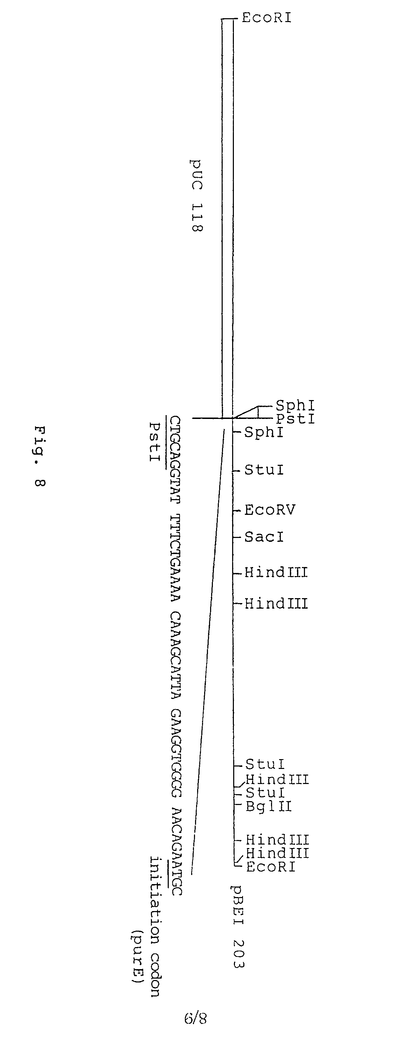

pBX118-9 (10 $\mu$g) obtained in Example 1 v) was digested with Kpn I and subjected to BAL31 digestion treatment for 30 seconds. Then, this was digested with Eco RI and fractionated by agarose gel electrophoresis to obtain DNA corresponding to about 3.6 kbp. This DNA was double-digested with Hin cII and Eco RI, ligated to pUC118 (0.5 $\mu$g) and used for transformation of Escherichia coli JM109 to select an

12

ampicillin resistant strain. As a result, the plasmid pBEI203 as shown in Fig. 8 was obtained.

iii) Construction of pDT32

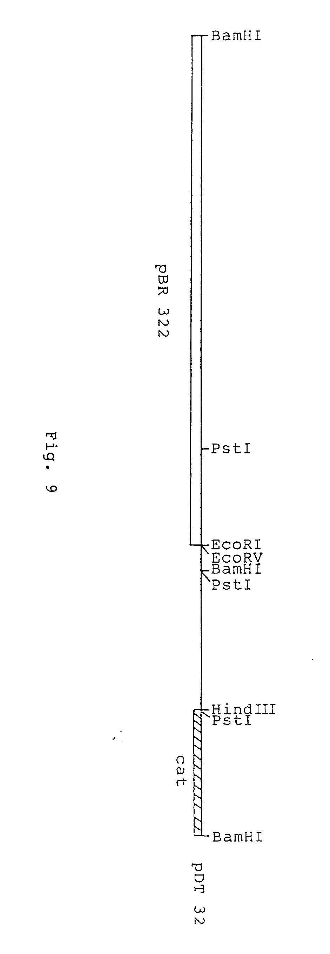

pSB22 (0.5 μg) obtained in the above i) was double-digested with Eco RV and Hin dIII and fractionated by agarose gel electrophoresis to recover a fragment (about 200 bp). This DNA was ligated to BLUESCRIPT SK cleaved with Eco RV and Hin dIII and used for transformation of Escherichia coli JM109. An ampicillin resistant strain was selected to obtain the plasmid pSK22. Then, pSK22 (0.5 μg) was double-digested with Eco RI and Pst I and fractionated by agarose gel electrophoresis to recover a fragment (about 220 bp). On the other hand, pBEI203 (0.5 μg) obtained in the above ii) was double-digested with Pst I and Hin dIII and fractionated by agarose gel electrophoresis to recover a fragment (about 1.2 kbp). Further, pCAT15 (0.5 μg) was double-digested with Hin dIII and Bam HI and fractionated by agarose gel electrophoresis to recover a fragment (about 1.0 kbp). Likewise, pBR322 (0.5 μg) was double-digested with Eco RI and Bam HI and fractionated by agarose gel electrophoresis to recover a fragment (about 3.9 kbp). These four fragments were mixed, ligated and used for transformation of Escherichia coli MM294 (ATCC 33625) to select an ampicillin resistant strain. As a result, the plasmid pDT32 as shown in Fig. 9 was obtained.

Example 4

i) PRPP amidotransferase activity of Bacillus subtilis 168 transformed with pDT32

Bacillus subtilis 168 was transformed with pDT32 (1 μg) obtained in Example 3 iii) to obtain chloramphenicol (10 μg/ml) resistant strains. One of these strains was cultivated with SPI medium, SPI medium containing adenine and SPI medium containing guanine, and PRPP amidotransferase activities were determined. As control experiments, the activities of Bacillus subtilis 168 was determined. The results are shown in Table 3.

Table 3

| Strains | SPI | SPI + adenine | SPI + guanine |
|---|---|---|---|
| B . subtilis 168 | 5.4 | 1.4 | 1.0 |
| B . subtilis 168 (pDT32) | 7.4 | 2.2 | 5.7 |
| (Units) | | | |

ii) Transformation of inosine-guanosine producer Bacillus subtilis BM1032 strain

Bacillus subtilis BM1032 strain was transformed with pDT32 (1 μg) obtained in Example 3 iii) to obtain chloramphenicol (10 μg/ml) resistant strains. One these strains was named as Bacillus subtilis BM2232 [IFO 15040, FERM BP-2937 (deposited May 28, 1990)].

iii) Productivity of inosine and guanosine by Bacillus subtilis BM2232 strain

Bacillus subtilis BM2232 strain obtained in the above ii) was cultivated according to the same manner as that described in Example 2 iii) and the amount of inosine and guanosine accumulated in the culture broth was measured. As a result, 13 mg/ml of inosine and 2 mg/ml of guanosine were accumulated. On the other hand, in the case of the control strain, Bacillus subtilis BM1032 strain, only 8.0 mg/ml of inosine and

13

1.1 mg/ml of guanosine were accumulated.


## Claims

1. Chromosomal DNA comprising a DNA sequence responsible for expression of a purine operon of a microorganism belonging to the genus Bacillus and DNA of the 3'-adjacent region of the sequence, a part or all of said DNA sequence responsible for expression of a purine operon being modified so as to increase the expression.

2. DNA according to claim 1, wherein the DNA sequence part to be modified is a DNA region concerned in repression by adenine related substances.

3. DNA according to claim 1, wherein the DNA sequence part to be modified is a DNA region concerned in repression by guanine related substances.

4. DNA according to claim 1, wherein the DNA sequence part to be modified is the promcter of a purine operon.

5. DNA according to claim 4, wherein the promoter of a purine operon is replaced with an expressible different promoter.

6. DNA according to claim 5, wherein the different promoter is that derived from chromosomal DNA of a microorganism belonging to the genus Bacillus or a phage of a microorganism belonging to the genus Bacillus .

7. A vector integrated with the DNA according to any one of claims 1 to 6.

8. A microorganism belonging to the genus Bacillus and being capable of producing inosine and/or guanosine which is transformed with the DNA according to any one of claims 1 to 7.

9. A microorganism according to claim 8 which is selected from the species Bacillus subtilis , Bacillus pumilus and Bacillus licheniformis .

10. A microorganism according to claim 8 or 9 which is selected from the strains Bacillus subtilis No. 115 [FERM BP-1327], Bacillus subtilis 168 [BGSC 1A1], Bacillus subtilis BM1032 [FERM BP-1242], Bacillus subtilis BM2032 [FERM BP-2536] and Bacillus subtilis BM 2232 [FERM BP-2937].

11. A process for producing inosine and/or guanosine which comprises cultivating a microorganism of the genus Bacillus according to any one of claims 8 to 10 in a culture medium to produce and accumulate inosine and/or guanosine and collecting the resultant.

Sequence Listing

SEQ ID NO: 1

SEQUENCE TYPE: Nucleotide

SEQUENCE LENGTH: 504 base pairs


STRANDEDNESS: double

TOPOLOGY: linear

MOLECULE TYPE: genomic DNA


ORIGINAL SOURCE

ORGANISM: *Bacillus subtilis*

STRAIN: FERM BP-1327


PROPERTIES: DNA fragment concerned with expression of

        purine operon


```
GATATCTGCC TGTAAGGCGA ATGGAAGAGC CTATTTTTAT GGAAAAAAAG GAAAATGGGT    60
CAATTCAGAT CGTTCCGTGC GGGAAAAAAA TCGTATTTGA AGGGAAATTG ATCTAAAACA   120
CGAACATTAG TAGAATGAAT TTTTGTATCG TTCGATAATA TCGTTGACAT TATCCATGTC   180
CGTTGTTAAG ATAAACATGA AATCAAAACA CGACCTCATA TAATCTTGGG AATATGGCCC   240
ATAAGTTTCT ACCCGGCAAC CGTAAATTGC CGGACTATGC AGGAAAGTGA TCGATAAAAC   300
TGACATGGAT ATATCGCAGA AGCGAACGAC TGACGATACA TGTACCATGC CCGGTTTGTA   360
TTGCTTCCTC ATAAGTGCAA TGCAGAGCGG GTATTTTTTA TTTTCTGAAA ACAAAAGCAT   420
TAGAAGGTGG GGAACAGA   ATG CAG CCG CTA GTA GGA ATC ATC ATG GGA AGC   471
ACT TCC GAT TGG GAG ACA ATG AAA CAC GCA TGC                         504
```

Sequence Listing

SEQ ID NO: 1

SEQUENCE TYPE: Nucleotide

SEQUENCE LENGTH: 504 base pairs


STRANDEDNESS: double

TOPOLOGY: linear

MOLECULE TYPE: genomic DNA


ORIGINAL SOURCE

ORGANISM: Bacillus subtilis

STRAIN: FERM BP-1327


PROPERTIES: DNA fragment concerned with expression of
            purine operon


```
GATATCTGCC TGTAAGGCGA ATGGAAGAGC CTATTTTTAT GGAAAAAAAG GAAAATGGGT    60
CAATTCAGAT CGTTCCGTGC GGGAAAAAAA TCGTATTTGA AGGGAAATTG ATCTAAAACA   120
CGAACATTAG TAGAATGAAT TTTTGTATCG TTCGATAATA TCGTTGACAT TATCCATGTC   180
CGTTGTTAAG ATAAACATGA AATCAAAACA CGACCTCATA TAATCTTGGG AATATGGCCC   240
ATAAGTTTCT ACCCGGCAAC CGTAAATTGC CGGACTATGC AGGAAAGTGA TCGATAAAAC   300
TGACATGGAT ATATCGCAGA AGCGAACGAC TGACGATACA TGTACCATGC CCGGTTTGTA   360
TTGCTTCCTC ATAAGTGCAA TGCAGAGCGG GTATTTTTTA TTTTCTGAAA ACAAAAGCAT   420
TAGAAGGTGG GGAACAGA   ATG CAG CCG CTA GTA GGA ATC ATC ATG GGA AGC   471
ACT TCC GAT TGG GAG ACA ATG AAA CAC GCA TGC                          504
```

SEQ ID NO: 2           .

SEQUENCE TYPE: Nucleotide

SEQUENCE LENGTH: 80 base pairs


STRANDEDNESS: double

TOPOLOGY: linear

MOLECULE TYPE: genomic DNA


ORIGINAL SOURCE

ORGANISM: Bacillus subtilis

STRAIN: FERM BP-1327


PROPERTIES: DNA fragment concerned with expression of

　　　　　　　purine operon


```
GGTACCGCGG GTATTTTTTA TTTTCTGAAA ACAAAAGCAT TAGAAGGTGG GGAACAGA       58
ATG CAG CCG CTA GTA GGA ATC A                                         80
```

SEQ ID NO: 3

SEQUENCE TYPE: Nucleotide

SEQUENCE LENGTH: 98 base pairs


STRANDEDNESS: double

TOPOLOGY: linear

MOLECULE TYPE: genomic DNA

PROPERTIES: promoter
　.

```
AATTCAAAAA TTTTGCAAAA AGTTGTTGAC TTTATCTACA AGGTGTGGCA TAATAATCTT      60

AATTGTGAGC GGATAACAAT TCTGCAGAAG CTTGAGCT                               98
```

SEQ ID NO: 4

SEQUENCE TYPE: Nucleotide

SEQUENCE LENGTH: 34 base pairs


STRANDEDNESS: double

TOPOLOGY: linear

MOLECULE TYPE: genomic DNA


ORIGINAL SOURCE

ORGANISM: <u>Bacillus</u> <u>subtilis</u>

STRAIN: FERM BP-1327


PROPERTIES: DNA fragment concerned with expression of

        purine operon

```
AAACATGAAA TCAAAACACG ACCTCAGGGG ATCC                                   34
```

SEQ ID NO: 5

SEQUENCE TYPE: Nucleotide

SEQUENCE LENGTH: 50 base pairs


STRANDEDNESS: double

TOPOLOGY: linear

MOLECULE TYPE: genomic DNA

ORIGINAL SOURCE

ORGANISM: _Bacillus_ _subtilis_

STRAIN: FERM BP-1327

PROPERTIES: DNA fragment concerned with expression of
            purine operon

CTGCAGGTAT TTTCTGAAAA CAAAGCATTA GAAGGTGGGG AACAGA ATG C    50

Cla I
Sph I
Stu I
EcoR V
Sac I
Hind III
Hind III

Stu I
Hind III
Stu I
Bgl II
Hind III
Hind III
EcoR I
Xba I
Hind III
Sph I
EcoR V
Cla I
EcoR I

Fig. 1

pBR 322

Sph I
Bam HI
Hind III —— EcoR V
Cla I

pPEC 32

20

Fig. 2

Fig. 3

```
     EcoR V    10         20         30         40         50
5'  GATATCTGCC TGTAAGGCGA ATGGAAGAGC CTATTTTTAT GGAAAAAAAG

         60         70         80         90        100
    GAAAATGGGT CAATTCAGAT CGTTCCGTGC GGGAAAAAAA TCGTATTTGA

        110        120        130        140        150
    AGGGAAATTG ATCTAAAACA CGAACATTAG TAGAATGAAT TTTTGTATCG

        160        170        180        190        200
    TTCGATAATA TCGTTGACAT TATCCATGTC CGTTGTTAAG ATAAACATGA
                   -35                         -10
        210        220        230        240        250
    AATCAAAACA CGACCTCATA TAATCTTGGG AATATGGCCC ATAAGTTTCT

        260        270        280        290        300
    ACCCGGCAAC CGTAAATTGC CGGACTATGC AGGAAAGTGA TCGATAAAAC

        310        320        330        340        350
    TGACATGGAT ATATCGCAGA AGCGAACGAC TGACGATACA TGTACCATGC

        360        370        380        390        400
    CCGGTTTGTA TTGCTTCCTC ATAAGTGCAA TGCAGAGCGG GTATTTTTTA

        410        420        430        440        450
    TTTTCTGAAA ACAAAAGCAT TAGAAGGTGG GGAACAGAAT GCAGCCGCTA
                                             initiation codon (purE)
        460        470        480        490        500
    GTAGGAATCA TCATGGGAAG CACTTCCGAT TGGGAGACAA TGAAACACGC
```

```
ATGC
─────
Sph I
```

Fig. 4

EP 0 412 688 A1

5'    AATT CAAAA ATTTT GCAAA AAGTT GTTGA CTTTA TCTAC AAGGT GTGGC

        GTTTT TAAAA CGTTT TTCAA CAACT GAAAT AGATG TTCCA CACCG


ATAAT AATCT TAATT GTGAG CGGAT AACAA TTCTG CAGAA GCTTG AGCT      3'

TATTA TTAGA ATTAA CACTC GCCTA TTGTT AAGAC GTCTT CGAAC


Fig. 5

Fig. 6

AAACATGAAA TCAAAACACG ACCTCAGGGGATCC

BamHI

SacI

EcoRV
HincII
BamHI
HincII
PstI
HindIII

pUC 118

SacI

pSB 22

Fig. 7

Fig. 8

EcoRI

pUC 118

SphI
PstI
SphI
StuI
EcoRV
SacI
HindIII
HindIII

StuI
HindIII
StuI
BglII
HindIII
HindIII
EcoRI

PstI

CTGCAGGTAT TTTCTGAAAA CAAAGCATTA GAAGGTGGGG AACAGAATGC

PstI

initiation codon (purE)

pBEI 203

6/8

27

Fig. 9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-273660 (TAKEDA CHEMICAL INDUSTRIES, LTD) <br> * page 2, line 1 - page 7, line 17 * | 1, 3, 7-9, 11 | C12N15/52 <br> C12P19/40 <br> C12N1/21 |
| X | EP-A-286303 (TAKEDA CHEMICAL INDUSTRIES, LTD) <br> * page 2, line 37 - page 8, line 39 * | 1-2, 7-11 | (C12N1/21, <br> C12R1:07) <br> (C12N1/21, |
| D,Y | JOURNAL OF BIOLOGICAL CHEMISTRY. <br> vol. 262, no. 17, 15 June 1987, BALTIMORE US <br> pages 8274 - 8287; EBBOLE, D.J. & ZALKIN, H.: <br> "Cloning and characterization of a 12-gene <br> cluster from Bacillus subtilis encoding nine <br> enzymes for de novo purine nucleotide synthesis" <br> * the whole document * | 1, 4-5, 7-9, 11 | C12R1:125) |
| Y | AGRICULTURAL AND BIOLOGICAL CHEMISTRY <br> vol. 52, no. 7, July 1988, TOKYO, JP <br> pages 1863 - 1865; MATSUI,K. et al.: <br> "Construction of tryptophan-producing <br> recombinant strains of Brevibacterium <br> lactofermentum using engineered trp operons" <br> * the whole document * | 1, 4-5, 7-9, 11 | |
| A | PATENT ABSTRACTS OF JAPAN <br> vol. 7, no. 281 (C-200)(1426) 15 December 1983, <br> & JP-A-58 158197 (AJINOMOTO K.K.) 20 September <br> 1983, <br> * the whole document * | 1-3 | TECHNICAL FIELDS SEARCHED (Int. Cl.5) <br><br> C12N <br> C12P |
| A | PATENT ABSTRACTS OF JAPAN <br> vol. 8, no. 7 (C-204)(1444) 12 January 1984, <br> & JP-A-58 175493 (AJINOMOTO K.K.) 14 October <br> 1983, <br> * the whole document * | 1-3 | |

The present search report has been drawn up for all claims

| Place of search | Date of completion of the search | Examiner |
|---|---|---|
| THE HAGUE | 06 NOVEMBER 1990 | ANDRES S.M. |